# EUROPÄISCHE PATENTANMELDUNG

(11) **EP 3 854 778 A1**
(43) Veröffentlichungstag der Anmeldung: **28.07.2021**
(21) Anmeldenummer: 20153906.1
(22) Anmeldetag: 27.01.2020
(51) Int. Cl.: C07C 67/39, C07C 69/06, C07C 53/02, C07C 51/23

(54) **VERFAHREN ZUR KATALYTISCHEN ERZEUGUNG EINES ALKYLFORMIATS**

(71) Anmelder: Friedrich-Alexander-Universität Erlangen-Nürnberg, 91054 Erlangen (DE); OxFA GmbH, 96110 Scheßlitz (DE)
(72) Erfinder: ALBERT, Jakob, 91054 Erlangen (DE); KUMPIDET, Chiraphat, 91052 Erlangen (DE)
(74) Vertreter: Dr. Gassner & Partner mbB

(57) **Zusammenfassung**

Die Erfindung betrifft ein Verfahren zur katalytischen Erzeugung eines Alkylformiats, wobei mindestens ein alpha-Hydroxyaldehyd, mindestens eine alpha-Hydroxycarbonsäure, mindestens ein Kohlenhydrat und/oder mindestens ein Glycosid mittels einer Vanadium in der Oxidationsstufe +IV oder +V enthaltenden Vanadium-Sauerstoff-Verbindung oder eines Salzes davon als Katalysator in einer Lösung umgesetzt wird, wobei die Lösung ein Alkanol enthält, wobei das dabei als Reaktionsprodukt entstehende Alkylformiat von mindestens einem dabei entstehenden weiteren Reaktionsprodukt separiert wird, wobei der bei der katalytischen Reaktion reduzierte Katalysator durch Oxidation wieder in seinen Ausgangszustand versetzt wird.

## Beschreibung

Die Erfindung betrifft ein Verfahren zur katalytischen Erzeugung eines Alkylformiats.

Bei dem Verfahren kann ein als Katalysator dienendes Polyoxometallat-Ion der allgemeinen Formel [PMoₓV_{y}O₄₀]ⁿ⁻ mit einem alpha-Hydroxyaldehyd, einer alpha-Hydroxycarbonsäure, einem Kohlenhydrat oder einem Glycosid in einer Lösung umgesetzt werden. Dabei ist 6 ≤ x ≤ 11, 1 ≤ y ≤ 6 und x + y = 12, wobei n, x und y jeweils eine ganze Zahl ist und 3 < n < 10 sein kann.

Ein Verfahren, bei welchem ein derartiger Katalysator mit einem alpha-Hydroxyaldehyd, einer alpha-Hydroxycarbonsäure, einem Kohlenhydrat oder einem Glycosid umgesetzt wird, ist aus der WO 2016/120169 bekannt. Das daraus bekannte Verfahren dient zur Erzeugung von Ameisensäure.

Aus Albert, Jakob, et al., Energy and Environmental Science 5 (2012), Seiten 7956 bis 7962 ist eine selektive Oxidation von Biomasse zu Ameisensäure unter Verwendung des Polyoxometallats H₅PV₂Mo₁₀O₄₀ als homogenen Katalysator, Sauerstoff als Oxidationsmittel, Wasser als Lösungsmittel und p-Toluolsulfonsäure als Additiv bekannt. Die beschriebene Oxidation erfolgte bei 90 °C und einem Sauerstoffpartialdruck von 30 bar. Es ist eine Ausbeute von bis zu 53% Ameisensäure nach 24 Stunden angegeben.

Aus Tang, Z. et al., ChemSusChem 2014, 7, Seiten 1557 bis 1567 ist eine durch Vanadyl-Kationen katalysierte Umsetzung von Zellulose zu Ameisensäure und Milchsäure bekannt. Insbesondere wird die Verwendung von VOSO₄ als Katalysator zur Umsetzung von Glucose zu Ameisensäure und zu Milchsäure offenbart. Durch die Bildung von CO₂ bei dieser Umsetzung ist die Ausbeute von Ameisensäure auf etwas über 50% beschränkt. Es wurde jedoch gefunden, dass das Zusetzen von Methanol oder Ethanol zu dem Reaktionssystem die Bildung von CO₂ während der Umsetzung von Glucose unter aeroben Bedingungen unterdrückt und die Ausbeute an Ameisensäure dadurch auf 70% bis 75% erhöht werden konnte.

Aus der EP 2 922 815 B1 ist ein Verfahren zur katalytischen Herstellung von Methylformiat durch Umsetzung von Methanol mit Kohlenmonoxid in Gegenwart eines Katalysatorsystems, das Alkaliformiat und Alkalialkoholat enthält, bekannt.

Aufgabe der vorliegenden Erfindung ist es, ein alternatives Verfahren zur Erzeugung von Alkylformiat anzugeben.

Die Aufgabe wird durch die Merkmale des Patentanspruchs 1 gelöst. Zweckmäßige Ausgestaltungen ergeben sich aus den Merkmalen der Patentansprüche 2 bis 15.

Erfindungsgemäß ist ein Verfahren zur katalytischen Erzeugung von Alkylformiat vorgesehen, wobei mindestens ein alpha-Hydroxyaldehyd, mindestens eine alpha-Hydroxycarbonsäure, mindestens ein Kohlenhydrat und/oder mindestens ein Glycosid als Substrat mittels einer Vanadium in der Oxidationsstufe +IV oder +V enthaltenden Vanadium-Sauerstoff-Verbindung oder eines Salzes davon als Katalysator in einer Lösung umgesetzt wird, wobei die Lösung ein Alkanol enthält. Das dabei als Reaktionsprodukt entstehende Alkylformiat wird von mindestens einem dabei entstehenden weiteren Reaktionsprodukt, wie beispielsweise Dimethoxyalkan, insbesondere Dimethoxymethan, separiert. Der bei der katalytischen Reaktion reduzierte Katalysator wird durch Oxidation wieder in seinen Ausgangszustand versetzt. Das Separieren von dem mindestens einen weiteren Reaktionsprodukt kann mittels bekannter Maßnahmen, wie Extraktion oder Verdampfen bzw. durch Destillation, erfolgen. Dabei kann entweder das Alkylformiat oder das weitere Reaktionsprodukt aus der Lösung abgesondert werden.

Der Katalysator kann ein Polyoxometallat-Ion der allgemeinen Formel [PMoₓV_{y}O₄₀]ⁿ⁻, wobei 6 ≤ x ≤ 11, 1 ≤ y ≤ 6 und x + y = 12, [WₓV_{y}O₁₉]ⁿ⁻, wobei x + y = 6, 3 ≤ x ≤ 5 und 1 ≤ y ≤ 3 oder [P₂WₓV_{y}O₆₂]ⁿ⁻, wobei x + y = 18, 12 ≤ x ≤ 17 und 1 ≤ y ≤ 6 oder ein VO²⁺ enthaltendes Salz, insbesondere VOSO₄, oder ein [VO₃]⁻ enthaltendes Salz, insbesondere NH₄VO₃, sein, wobei n, x und y jeweils eine ganze Zahl ist. Der Wert von n ergibt sich dabei aus den Teilladungen der im Katalysator enthaltenen Elemente. Bei [PMoₓV_{y}O₄₀]ⁿ⁻ beispielsweise ist 3 < n < 10. Als gut geeignet hat sich das Polyoxometallat-Ion [PMoₓV_{y}O₄₀]ⁿ⁻, insbesondere [PMo₇V₅O₄₀]⁸⁻ (HPA-5), erwiesen. Wegen der von den Ionen gebildeten spezifischen Strukturen wird [PMoₓV_{y}O₄₀]ⁿ⁻ auch als Keggin-Ion, [WₓV_{y}O₁₉]ⁿ⁻ als Lindqvist-Ion und [P₂WₓV_{y}O₆₂]ⁿ⁻ als Wells-Dawson-Ion bezeichnet.

Bei dem Substrat kann es sich jeweils um ein alpha-Hydroxyaldehyd, eine alpha-Hydroxycarbonsäure, ein Kohlenhydrat oder ein Glycosid oder eine beliebige Mischung aus jeweils einem oder mehreren alpha-Hydroxyaldehyd(en), einer oder mehreren alpha-Hydroxycarbonsäure(n), einem oder mehreren Kohlenhydrat(en) und/oder einem oder mehreren Glycosid(en) handeln.

Die Erfinder haben nicht nur festgestellt, dass der oben genannte Katalysator und die oben genannten Substrate zur Bildung von Alkylformiat führen, sondern auch, dass die Gegenwart eines Alkanols in der Lösung bewirkt, dass eine Totaloxidation von Teilen des Substrats zu CO₂ und H₂O abhängig vom Alkanolanteil reduziert oder sogar vollständig unterbunden wird. Die Lösung kann neben dem Alkanol mindestens ein damit mischbares weiteres Lösungsmittel enthalten oder nur aus dem Alkanol oder einer Mischung von Alkanolen bestehen. Das weitere Lösungsmittel kann Wasser sein.

Wenn die Lösung neben dem Alkanol auch Wasser enthält, wird neben dem Alkylformiat auch Ameisensäure als das weitere Reaktionsprodukt gebildet. Die Ausbeute an Ameisensäure ist in Gegenwart des Alkanols deutlich höher, als in rein wässriger Lösung.

Das Alkanol kann ein unverzweigtes Alkanol, d. h. ein n-Alkanol, sein und/oder 1 bis 4 Kohlenstoffatome umfassen. Das Alkanol kann Methanol, Ethanol, n-Propanol oder n-Butanol sein. Insbesondere kann es sich bei dem Alkanol um Methanol handeln. Methanol ist wegen des daraus gebildeten Methylformiats vorteilhaft, weil Methylformiat einen sehr niedrigen Siedepunkt von nur 32 °C aufweist. Es kann daher einfach von dem in Gegenwart von Wasser in der Lösung entstehenden Gemisch aus Ameisensäure und Methylformiat ohne Hilfsstoffe und ohne die Bildung eines Azeotrops durch Verdampfen abgesondert bzw. durch Destillation gewonnen werden. Ethanol, n-Propanol oder n-Butanol weisen allerdings nicht die Giftigkeit von Methanol auf und sind wegen der daraus resultierenden geringeren Arbeitsschutzmaßnahmen günstiger zu handhaben. Die aus Ethanol und n-Propanol resultierenden Alkylformiate Ethylformiat und n-Propylformiat weisen ebenfalls Siedepunkte auf, welche unterhalb des Siedepunkts der Ameisensäure von 101 °C liegen. Auch diese Alkylformiate lassen sich durch Verdampfen bzw. Destillation von dem in Gegenwart von Wasser in der Lösung entstehenden Gemisch aus Ameisensäure und Alkylformiat absondern.

Durch das Absondern des gebildeten Alkylformiats aus der Lösung erlaubt das Verfahren auch eine günstige und hochselektive Herstellung von Ameisensäure mit einer deutlich höheren Ausbeute als bei einer Umsetzung des jeweiligen Substrats in Abwesenheit eines Alkanols. Es konnten Selektivitäten im Hinblick auf die Bildung von Alkylformiat und/oder Ameisensäure von über 90% erreicht werden. Dies war auch ohne die Verwendung eines zusätzlichen Reaktionsbeschleunigers oder Extraktionsmittels möglich. Die Erfinder haben erkannt, dass der Anteil an bei der Reaktion erzeugtem Alkylformiat umso größer ist, je höher der Anteil an Alkanol in der Lösung ist. Wenn das Lösungsmittel ausschließlich aus dem Alkanol besteht, entsteht bei der Umsetzung des Substrats keine Ameisensäure. Umgekehrt kann der Anteil an der bei der Reaktion erzeugten Ameisensäure durch eine Erhöhung des Wasseranteils in der Lösung gesteigert werden. Die Erhöhung der Selektivität der Reaktion in Bezug auf die Bildung des Alkylformiats und der Ameisensäure und die Reduzierung der Bildung von CO₂ bei der Reaktion ist jedoch auch schon bei einer geringen Menge an Alkanol in der, insbesondere Wasser enthaltenden, Lösung feststellbar.

Bei einer Ausgestaltung des Verfahrens enthält die Lösung zumindest 5 Gew.-% Alkanol, insbesondere zumindest 10 Gew.-% Alkanol, insbesondere zumindest 20 Gew.-% Alkanol, insbesondere zumindest 30 Gew.-% Alkanol, insbesondere zumindest 50 Gew.-% Alkanol, insbesondere zumindest 70 Gew.-% Alkanol, insbesondere zumindest 80 Gew.-% Alkanol, insbesondere zumindest 90 Gew.-% Alkanol, insbesondere zumindest 95 Gew.-% Alkanol, insbesondere 100 Gew.-% Alkanol.

Die Lösung kann neben dem Alkanol mindestens ein weiteres nichtwässriges Lösungsmittel enthalten. Auch die Anwesenheit des Weiteren nichtwässrigen Lösungsmittels anstelle von Wasser erhöht die genannte Selektivität und vermindert die Entstehung von CO₂.

Bei einer Ausgestaltung des Verfahrens wird der reduzierte Katalysator durch Oxidation mittels Sauerstoff oder einem Sauerstoff enthaltenden Oxidationsmittel wieder in seinen Ausgangszustand versetzt. Die Oxidation mittels Sauerstoff kann eine Oxidation mittels molekularem Sauerstoff als reines Gas oder in einem den molekularen Sauerstoff enthaltenden Gasgemisch, beispielsweise Luft oder synthetische Luft, sein. Bei synthetischer Luft handelt es sich im Allgemeinen um ein aus Sauerstoff und Stickstoff bestehendes Gasgemisch, bei welchem der Sauerstoffanteil im Bereich von 19,5 Vol.-% bis 21,5 Vol.-% liegt.

Das Sauerstoff enthaltende Oxidationsmittel kann ein Peroxid, insbesondere H₂O₂, oder N₂O sein.

Die Oxidation mittels dem molekularen Sauerstoff kann bei einem Sauerstoffdruck - im Falle von Sauerstoff als reines Gas - oder einem Sauerstoffpartialdruck - im Falle eines Gasgemischs - im Bereich von 1 bar bis 250 bar, insbesondere 1 bar bis 120 bar, insbesondere 1 bar bis 80 bar, insbesondere 1 bar bis 50 bar, insbesondere 1 bar bis 30 bar, insbesondere 5 bar bis 20 bar, insbesondere 5 bar bis 10 bar, erfolgen. Zur Oxidation kann die Lösung, beispielsweise in einem statischen Mischer oder durch heftiges Rühren, mit dem molekularen Sauerstoff beaufschlagt werden. Für das Verfahren hat es sich als günstig und effizient erwiesen, wenn das Umsetzen des mindestens einen alpha-Hydroxyaldehyds, der mindestens einen alpha-Hydroxycarbonsäure, des mindestens einen Kohlenhydrats und/oder des mindestens einen Glycosids mittels des Katalysators bei einer Temperatur von höchstens 150 °C, insbesondere höchstens 120 °C, insbesondere in einem Bereich von 65 °C bis 120 °C, insbesondere in einem Bereich von 70 °C bis 100 °C, insbesondere in einem Bereich von 70 °C bis 90 °C, erfolgt.

Bei der alpha-Hydroxycarbonsäure kann es sich um Glycolsäure oder Milchsäure und bei dem Kohlenhydrat um ein Monosaccharid, insbesondere mit 5 oder 6 Kohlenstoffatomen, ein Disaccharid, insbesondere mit 12 Kohlenstoffatomen, ein Oligosaccharid oder ein Polysaccharid handeln. Das Monosaccharid kann eine Aldose, insbesondere Glucose oder Xylose, sein. Bei dem Disaccharid kann es sich um Saccharose oder Cellobiose handeln. Das Oligosaccharid kann ein Heterooligosaccharid sein. Bei dem Polysaccharid kann es sich um Stärke, Zellulose, Hemizellulose oder ein Heteropolysaccharid, insbesondere ein Xylan, handeln.

Bei einem Ausführungsbeispiel ist/sind das mindestens eine alpha-Hydroxyaldehyd, die mindestens eine alpha-Hydroxycarbonsäure, das mindestens eine Kohlenhydrat und/oder das mindestens eine Glycosid in einem, insbesondere nachwachsenden, Rohstoff oder einem nach einer Umsetzung des Rohstoffs daraus hervorgehenden Reststoff enthalten. Der Rohstoff kann ein Stoff biologischer Herkunft, insbesondere pflanzlicher Herkunft, sein. Bei dem Rohstoff kann es sich um Lignocellulose enthaltende Biomasse, wie beispielsweise verholztes Pflanzenmaterial oder Sägemehl, handeln. Der Rohstoff kann ein unbehandelter, d. h. nicht chemisch aufgeschlossener, Rohstoff sein. Durch den chemischen Aufschluss können den Katalysator inaktivierende Chemikalien in dem Rohstoff enthalten sein. Bei dem Reststoff oder dem nachwachsenden Rohstoff kann es sich um eine Pflanze, einen Pilz oder Bakterien oder Bestandteile von Pflanzen, Pilzen oder Bakterien, Holz, insbesondere in Form von Holzmehl oder Holzspänen, Papier, insbesondere Altpapier, Algen, Cyanobakterien oder Silage handeln. Das alpha-Hydroxyaldehyd, die alpha-Hydroxycarbonsäure, das Kohlenhydrat oder das Glycosid kann auch ein Gemisch aus mindestens zwei der genannten Substanzen umfassen oder aus mindestens einer der genannten Substanzen oder dem Gemisch entstanden sein, wie dies z. B. bei Braunkohle oder Torf der Fall ist.

Nachfolgend wird die Erfindung anhand von Ausführungsbeispielen näher erläutert.

In einem ersten Ausführungsbeispiel wurden jeweils in 10 g Lösungsmittel, welches aus Wasser und/oder Methanol in unterschiedlichen Gewichtsanteilen bestand, 1 mmol Glucose gelöst und 0,1 mmol des Polyoxometallat-Ions [PMo₇V₅O₄₀]⁸⁻ (= HPA-5) zugesetzt. Diese Lösung wurde für 24 Stunden mit tausend Umdrehungen pro Minute gerührt und dabei bei einer Temperatur von 90 °C gehalten und Sauerstoff mit einem Sauerstoffpartialdruck von 20 bar ausgesetzt. Die Ergebnisse sind in der nachfolgenden Tabelle 1 zusammengefasst:

**Tabelle 1:**

| Substrat | w_{H₂O,vor} / Gew.-% | w_{H₂O,nach} / Gew.-% | X_{Glu} / % (HPLC) | DME | FAI | MM | DMM | Verhältnis FA:MF | Y_{CO₂/CO} /% |
|---|---|---|---|---|---|---|---|---|---|
| Glucose | 0 | 4,4 | 100 | - | - | - | x | 0:100 | -/- |
| Glucose | 10 | 14,0 | 100 | - | - | x | x | 20:80 | 0,5/- |
| Glucose | 20 | | 99 | - | - | x | x | 25:75 | 0,6/- |
| Glucose | 30 | | 99 | - | - | x | x | 35:65 | 1,0/0,2 |
| Glucose | 40 | | 98 | - | - | x | x | 42:58 | 1,2/0,2 |
| Glucose | 48 | | 98 | - | - | x | x | 44:56 | 1,8/0,3 |
| Glucose | 49 | | 98 | - | - | x | x | 46:54 | 1,6/0,2 |
| Glucose | 70 | | 97 | - | - | x | x | 61:39 | 2,2/0,2 |
| Glucose | 90 | | 93 | - | - | x | x | 81:19 | 3,3/0,3 |
| Glucose | 100 | | 100 | - | - | - | - | 100:0 | 27,4/0,4 |

Die Spalte "w_{H₂O,vor} / Gew.-%" gibt den gewichtsprozentualen Anteil an Wasser in der Lösung vor der Reaktion an. Beträgt dieser Anteil 0 Gew.-%, besteht die Lösung ausschließlich aus Methanol. Beträgt dieser Anteil 100 Gew.-%, ist in der Lösung kein Methanol enthalten. Die Spalte "w_{H₂O,nach}/ Gew.-%" gibt den gewichtsprozentualen Anteil an Wasser in der Reaktionslösung nach der Reaktion an. "X_{Glu} / % (HPLC)" gibt den mittels Hochleistungsflüssigkeitschromatographie (HPLC) bestimmten prozentualen Umsatz der eingesetzten Glucose an. In der obigen Tabelle 1 haben die Abkürzungen die folgenden Bedeutungen:

| | |
|---|---|
| DME: | Dimethylether |
| FAI: | Formaldehyd |
| MM: | Methoxymethanol |
| DMM: | Dimethoxymethan |
| FA: | Ameisensäure |
| MF: | Methylformiat |

Das Vorhandensein der Reaktionsprodukte DME, FAI, MM und DMM wurde mittels ¹³C-NMR-Spektroskopie bestimmt. Das Vorhandensein von FA und MF wurde ebenfalls mittels ¹³C-NMR-Spektroskopie und das in der Spalte "FA:MF" angegebene Mengenverhältnis von Ameisensäure zu Methylformiat durch die Bestimmung des Verhältnisses der Peakflächen für Ameisensäure und Methylformiat im ¹³C-NMR-Spektrum bestimmt. In der Spalte "Y_{CO₂/CO} / %" sind die mittels Gaschromatographie bestimmten und auf die jeweils eingesetzte Glucose bezogenen prozentualen Ausbeuten an CO₂ und CO angegeben. Sofern kein Reaktionsprodukt aufgefunden wurde, wurde dies mit einem "-" gekennzeichnet, ansonsten mit einem "x".

Aus Tabelle 1 ist ersichtlich, dass bei einem anfänglichen Wassergehalt von 0 Gew.-%, d. h. 100 Gew.-% Methanol, kein CO₂, kein CO und keine Ameisensäure entstanden ist und demnach das Verhältnis FA:MF 0:100 beträgt. Bei sämtlichen Reaktionen, in welchen das Lösungsmittel Methanol enthielt, ist Dimethoxymethan als Nebenprodukt entstanden. Als Nebenprodukt bei allen Reaktionen, die anfangs sowohl Wasser als auch Methanol in der Lösung enthielten, ist Methoxymethanol entstanden. Ansonsten zeigt Tabelle 1, dass der Anteil des entstandenen Methylformiats umso höher war, je größer der Anteil an Methanol in der Lösung war. Umgekehrt war der Anteil der entstandenen Ameisensäure umso höher, je größer der Anteil an Wasser in der Lösung war. Bei Abwesenheit von Methanol sind nur Ameisensäure, CO₂ und CO entstanden. Methylformiat ist dabei nicht entstanden. Tabelle 1 zeigt in der Spalte "Y_{CO₂/CO} / %" auch, dass in Abwesenheit von Methanol ein verhältnismäßig hoher Anteil an CO₂ entsteht, jedoch bereits 10% Methanol ausreichen, um die CO₂- und CO-Entstehung stark zu reduzieren. Die Tabelle zeigt auch, dass nur der am Anfang der Reaktion vorhandene Wassergehalt entscheidend für die Reaktionsprodukte ist. Das bei der Reaktion in 100 Gew.-% Methanol entstehende Wasser bewirkt keine Entstehung von Ameisensäure.

In einem zweiten Ausführungsbeispiel wurden jeweils in 10 g Methanol als Lösungsmittel 1 mmol Glucose gelöst und 0,5 mmol von in einem Katalysator enthaltenem Vanadium zugesetzt. Als Katalysatoren wurden das Polyoxometallat-Ion [PMo₇V₅O₄₀]⁸⁻ (= HPA-5), VOSO₄, NH₄VO₃ und K₅V₃W₃O₁₉ eingesetzt. Diese Lösung wurde für 24 Stunden mit tausend Umdrehungen pro Minute gerührt und dabei bei einer Temperatur von 90 °C gehalten und Sauerstoff mit einem Sauerstoffpartialdruck von 20 bar ausgesetzt. Die Ergebnisse sind in den nachfolgenden Tabelle 2 zusammengefasst:

**Tabelle 2:**

| Substrat | Katalysator | w_{H₂O,nach} / Gew.-% | DME | FAI | MM | DMM | FA | MF | Y_{CO₂/CO} /% |
|---|---|---|---|---|---|---|---|---|---|
| ohne | HPA-5 | 2,1 | x | - | - | x | - | - | -/- |
| Glucose | HPA-5 | 4,4 | - | - | (x) | x | - | x | -/- |
| Glucose | VOSO₄ | 4,2 | - | - | (x) | x | - | x | 0,6/- |
| Glucose | NH₄VO₃ | 2,9 | - | - | x | (x) | - | x | 1,8/0,2 |
| Glucose | K₅V₃W₃O₁₉ | 2,1 | - | - | x | (x) | - | x | 1,3/0,2 |

Die Abkürzungen haben die oben für Tabelle 1 angegebenen Bedeutungen. Die Spalte "w_{H₂O,nach}/ Gew.-%" gibt den gewichtsprozentualen Anteil an Wasser in der Reaktionslösung nach der Reaktion an. In der Spalte "Y_{CO₂/CO} / %" sind die mittels Gaschromatographie bestimmten und auf die jeweils eingesetzte Glucose bezogenen prozentualen Ausbeuten an CO₂ und CO angegeben. Sofern kein Reaktionsprodukt aufgefunden wurde, wurde dies mit einem "-" gekennzeichnet, ansonsten mit einem "x". "(x)" bedeutet, dass nur Spuren des jeweiligen Reaktionsprodukts aufgefunden wurden.

Tabelle 2 zeigt, dass verschiedene Vanadium in der Oxidationsstufe +IV oder +V enthaltende Vanadium-Sauerstoff-Verbindungen bzw. Salze davon als Katalysator zur Erzeugung des Alkylformiats eingesetzt werden können. Tabelle 2 bestätigt weiterhin, dass bei anfänglicher Abwesenheit von Wasser keine Ameisensäure entsteht, selbst wenn im Laufe der Reaktion Wasser entsteht.

In einem dritten Ausführungsbeispiel wurden in 100 g Methanol als Lösungsmittel jeweils Buchenspäne suspendiert oder 68 gewichtsprozentige Melasse gelöst und 1 mmol des Polyoxometallat-Ions [PMo₇V₅O₄₀]⁸⁻ (= HPA-5) als Katalysator zugesetzt. Diese Suspension bzw. Lösung wurde für 24 Stunden mit tausend Umdrehungen pro Minute gerührt und dabei bei einer Temperatur von 90 °C gehalten und Sauerstoff mit einem Sauerstoffpartialdruck von 20 bar ausgesetzt. Die Ergebnisse sind in den nachfolgenden Tabelle 3 zusammengefasst:

**Tabelle 3:**

| Substrat | w_{H₂O,Substrat} / Gew.-% | w_{H₂O,nach} / Gew.-% | DME | FAI | MM | DMM | FA | MF | Y_{CO₂/CO} / % |
|---|---|---|---|---|---|---|---|---|---|
| Buchenspäne | | 2,7 | x | - | x | x | - | x | -/- |
| Melasse (68 Gew.-%ig) | 31,8 w_{H₂O,vor} = 0,13 | 2,0 | x | - | - | x | - | x | 3,6/- |

Die Abkürzungen haben die oben für die Tabellen 1 und 2 angegebenen Bedeutungen. Die Spalte "w_{H₂O,Substrat} / Gew.-%" gibt den gewichtsprozentualen Anteil an Wasser im Substrat an. "w_{H₂O,vor} / Gew.-%" gibt den gewichtsprozentualen Anteil an Wasser in der Lösung vor der Reaktion an. Die Spalte "w_{H₂O,nach}/ Gew.-%" gibt den gewichtsprozentualen Anteil an Wasser in der Reaktionslösung nach der Reaktion an. Sofern kein Reaktionsprodukt aufgefunden wurde, wurde dies mit einem "-" gekennzeichnet, ansonsten mit einem "x". Von jedem der eingesetzten Substrate wurde vor der Reaktion eine organische (CHNS)-Elementaranalyse durchgeführt, um in jedem der Substrate jeweils den gewichtsprozentualen Anteil an Kohlenstoff, Wasserstoff, Stickstoff und Schwefel sowie aus der Differenz zu 100 % den gewichtsprozentualen Anteil an Sauerstoff zu bestimmen. Gewichtet mit den molaren Massen ergibt sich daraus der jeweilige Stoffmengenanteil, auf dessen Grundlage die in der Spalte "Y_{CO₂/CO} / %" angegebene prozentuale Ausbeute an CO₂ bestimmt wurde. Eine Bildung von CO konnte für keines der Substrate nachgewiesen werden. Für Buchenspäne als Substrat konnte auch keine CO₂-Bildung nachgewiesen werden.

Tabelle 3 zeigt, dass auch andere Substrate als Glucose eingesetzt werden können. Hier nicht gezeigt, jedoch von den Erfindern nachgewiesen wurde auch bei diesen Substraten die Entstehung von Ameisensäure, sobald die Lösung neben dem Alkanol zu Beginn der Reaktion auch Wasser enthielt.

## Patentansprüche

1. Verfahren zur katalytischen Erzeugung eines Alkylformiats, wobei mindestens ein alpha-Hydroxyaldehyd, mindestens eine alpha-Hydroxycarbonsäure, mindestens ein Kohlenhydrat und/oder mindestens ein Glycosid mittels einer Vanadium in der Oxidationsstufe +IV oder +V enthaltenden Vanadium-Sauerstoff-Verbindung oder eines Salzes davon als Katalysator in einer Lösung umgesetzt wird, wobei die Lösung ein Alkanol enthält, wobei das dabei als Reaktionsprodukt entstehende Alkylformiat von mindestens einem dabei entstehenden weiteren Reaktionsprodukt separiert wird, wobei der bei der katalytischen Reaktion reduzierte Katalysator durch Oxidation wieder in seinen Ausgangszustand versetzt wird.

2. Verfahren nach Anspruch 1, wobei der Katalysator ein Polyoxometallat-Ion der allgemeinen Formel [PMoₓV_{y}O₄₀]ⁿ⁻, wobei 6 ≤ x ≤ 11, 1 ≤ y ≤ 6 und x + y = 12, [WₓV_{y}O₁₉]ⁿ⁻, wobei x + y = 6, 3 ≤ x ≤ 5 und 1 ≤ y ≤ 3 oder [P₂WₓV_{y}O₆₂]ⁿ⁻, wobei x + y = 18, 12 ≤ x ≤ 17 und 1 ≤ y ≤ 6 oder ein VO²⁺ enthaltendes Salz, insbesondere VOSO₄, oder ein [VO₃]⁻ enthaltendes Salz, insbesondere NH₄VO₃, ist, wobei n, x und y jeweils eine ganze Zahl ist.

3. Verfahren nach Anspruch 1 oder 2, wobei der Katalysator [PMo₇V₅O₄₀]⁸⁻ ist.

4. Verfahren nach einem der vorhergehenden Ansprüche, wobei das Alkanol ein n-Alkanol ist und/oder 1 bis 4 Kohlenstoffatome umfasst.

5. Verfahren nach einem der vorhergehenden Ansprüche, wobei das Alkanol Methanol, Ethanol, n-Propanol oder n-Butanol, insbesondere Methanol, ist.

6. Verfahren nach einem der vorhergehenden Ansprüche, wobei die Lösung neben dem Alkanol auch Wasser enthält und dadurch neben dem Alkylformiat Ameisensäure als das weitere Reaktionsprodukt gebildet wird.

7. Verfahren nach einem der vorhergehenden Ansprüche, wobei die Lösung zumindest 10 Gew.-% Alkanol, insbesondere zumindest 50 Gew.-% Alkanol, enthält.

8. Verfahren nach einem der vorhergehenden Ansprüche, wobei die Lösung zumindest 90 Gew.-% Alkanol, insbesondere zumindest 95 Gew.-% Alkanol, enthält.

9. Verfahren nach einem der vorhergehenden Ansprüche, wobei der reduzierte Katalysator durch Oxidation mittels Sauerstoff oder einem Sauerstoff enthaltenden Oxidationsmittel wieder in seinen Ausgangszustand versetzt wird.

10. Verfahren nach Anspruch 9, wobei die Oxidation mittels dem Sauerstoff eine Oxidation mittels molekularem Sauerstoff als reines Gas oder in einem den molekularen Sauerstoff enthaltenden Gasgemisch, insbesondere Luft, ist und das den Sauerstoff enthaltende Oxidationsmittel ein Peroxid, insbesondere H₂O₂, oder N₂O ist.

11. Verfahren nach Anspruch 10, wobei die Oxidation mittels dem molekularen Sauerstoff bei einem Sauerstoffdruck oder Sauerstoffpartialdruck im Bereich von 1 bar bis 50 bar, insbesondere 5 bar bis 10 bar, erfolgt.

12. Verfahren nach einem der vorhergehenden Ansprüche, wobei das Umsetzen des mindestens einen alpha-Hydroxyaldehyds, der mindestens einen alpha-Hydroxycarbonsäure, des mindestens einen Kohlenhydrats und/oder des mindestens einen Glycosids mittels des Katalysators bei einer Temperatur von höchstens 150 °C, insbesondere in einem Bereich von 70 °C bis 100 °C, erfolgt.

13. Verfahren nach einem der vorhergehenden Ansprüche, wobei es sich bei der alpha-Hydroxycarbonsäure um Glycolsäure oder Milchsäure und bei dem Kohlenhydrat um ein Monosaccharid, insbesondere mit 5 oder 6 Kohlenstoffatomen, ein Disaccharid, insbesondere mit 12 Kohlenstoffatomen, ein Oligosaccharid oder ein Polysaccharid handelt.

14. Verfahren nach Anspruch 13, wobei es sich bei dem Monosaccharid um eine Aldose, insbesondere Glucose oder Xylose, bei dem Disaccharid um Saccharose oder Cellobiose, bei dem Oligosaccharid um ein Heterooligosaccharid und bei dem Polysaccharid um Stärke, Zellulose, Hemizellulose oder ein Heteropolysaccharid, insbesondere ein Xylan, handelt.

15. Verfahren nach einem der vorherigen Ansprüche, wobei das mindestens eine alpha-Hydroxyaldehyd, die mindestens eine alpha-Hydroxycarbonsäure, das mindestens eine Kohlenhydrat und/oder das mindestens eine Glycosid in einem, insbesondere nachwachsenden, Rohstoff oder einem nach einer Umsetzung des Rohstoffs daraus hervorgehenden Reststoff enthalten ist/sind.
